(19) Europäisches Patentamt
European Patent Office
Office européen des brevets

(11) EP 1 923 048 B1

(12) EUROPÄISCHE PATENTSCHRIFT

(45) Veröffentlichungstag und Bekanntmachung des Hinweises auf die Patenterteilung:
**10.08.2011 Patentblatt 2011/32**

(51) Int Cl.:
***A61K 8/97*** *(2006.01)* ***A61Q 5/10*** *(2006.01)*
***A61K 8/22*** *(2006.01)*

(21) Anmeldenummer: **07020098.5**

(22) Anmeldetag: **13.10.2007**

(54) **Haarbehandlung zur verringerung der Haarschädigung**

Hair treatment to minimise hair damage

Traitement capillaire en vue de la réduction de l'endommagement des cheveux

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **10.11.2006 DE 102006053344**

(43) Veröffentlichungstag der Anmeldung:
**21.05.2008 Patentblatt 2008/21**

(73) Patentinhaber: **Henkel AG & Co. KGaA**
**40589 Düsseldorf (DE)**

(72) Erfinder:
- **Döring, Thomas**
  **41542 Dormagen (DE)**
- **Kainz, Sabine**
  **47447 Moers (DE)**
- **Reichert, Anja**
  **40629 Düsseldorf (DE)**
- **Hollenberg, Detlef**
  **40699 Erkrath (DE)**

(56) Entgegenhaltungen:
**EP-A- 1 174 121** **WO-A-2004/050047**
**DE-A1- 10 229 995** **FR-A- 2 841 782**
**KR-B1- 100 625 180** **US-A1- 2006 198 810**

- **DATABASE TCM SIPO; "The chinese medicinal biological cosmetics for wrinkle eliminating and skin care and its preparation method" XP002468023**
- **DATABASE WPI Week 200382 Derwent Publications Ltd., London, GB; AN 2003-893970 XP002467941 & KR 2003 064 004 A (KIM Y H) 31. Juli 2003 (2003-07-31)**
- **DATABASE WPI Week 199702 Derwent Publications Ltd., London, GB; AN 1997-017313 XP002467942 & JP 08 283172 A (KOSE KK) 29. Oktober 1996 (1996-10-29)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die kosmetische Verwendung einer Kombination ausgewählter Pflanzenextrakte mit α-Lipensäure, insbesondere während der oxidativen Haarbehandlung, zur Verringerung der Haarschadigung. Ein entsprechendes Verfahren zur insbesondere oxidativen Haarbehandlung, sowie die darin verwendbaren Mittel sind ebenso Gegenstand der vorliegenden Erfindung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Die permanente, oxidative Färbung sowie die Haarbleiche und die Fixierung im Rahmen der Dauerwelle finden meist als oxidative Haarbehandlung In Gegenwart von Oxidationsmitteln, wie Wasserstoffperoxyd, statt. Das Oxidationsmittel erzielt hierbei leider nicht nur die erwünschte kosmetische Wirkung, sondern schädigt zusätzlich die Struktur des Haarkeratins. Das keratineigene Cystin oxidiert dabei zur Cysteinsäure, was sich negativ auf die Stabilität, die Haptik und die Optik der Haarfaser auswirkt. Derart geschädigtes Haar wirkt stumpf und spröde. In extremen Fällen kommt es sogar zum Haarbruch.

Darüber hinaus kommt während der oxidativen Haarbehandlung die Kopfhaut mit dem Oxidationsmittel in Berührung, Aus diesem Grunde spürt der empfindliche Proband während der oxidativen Haarbehandlung oftmals ein Missempfinden auf der Kopfhaut. Dieses Missempfinden wird meist als Stechen oder Jucken beschrieben.

Dokument DE 102 29 995 A1 offenbart eine kosmetische und dermatologische Zubereitungen mit einem Gehalt an alpha-Liponsäure. Sie wird zur Behandlung von Haut und Haaren, sowie zum Schutz gegen oxidationsbedingte Abbaureaktionen, verwendet.

Es ist die Aufgabe der vorliegenden Erfindung, die zuvor beschriebenen Nebenwirkungen von Haarbehandlungsmitteln Insbesondere nach aber bevorzugt bereits während einer oxidativen Haarbehandlung, zu verringern, ohne den Wirkungsgrad des Haarbehandlungsmittels, insbesondere bezüglich Farbintensität, Farbechtheit, Aufhellleistung bzw. Wellwirkung, zu verschlechtern.

Überraschenderweise wurde nun gefunden, daß die Aufgabe in hervorragendem Maße durch die Verwendung einer spezifischen Kombination von Pflanzenextrakten mit α-Liponsäure gelöst wird. Die Wirkung entfaltet sich insbesondere während der oxidativen Haarbehandlung.

Die Pflanzenextrakte aus der Wurzel von *Puearia lobata* und aus der Blüte und/oder aus den Knospen der *Sophora japonica L.* sind aus der chinesischen Heilmedizin bekannt.

Ein erster Gegenstand der vorliegenden Erfindung ist die kosmetische Verwendung einer Pflanzenextraktkombination aus

- Extrakt aus der Wurzel von *Pueraria lobata* und
- Extrakt aus der Blüte und/oder aus den Knospen der *Sophora japonica L.* mit α-Liponsäure in Form der freien Säure und/oder der Salzform zur Haarpflege, insbesondere zur Verminderung der oxidativen Haarschädigung.

Die Extrakte aus den oben angegebenen Teilen der besagten Pflanzen werden üblicherweise durch Extraktion mit Wasser, organischen Lösemittel, wie z.B. bei Raumtemperatur flüssige Kohlenwasserstoffe, Ethern oder Alkoholen, sowie deren Mischungen bereitgestellt. Bevorzugte Extraktionsmittel sind Wasser und/oder Alkohole. Unter den Alkoholen sind dabei ($C_1$ bis $C_6$)-Alkohole, wie Ethanol und Isopropanol, insbesondere aber mehrwertige Alkohole wie Ethylenglykol, Propylenglykol und Butylenglykol und zwar sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen. Die Wasserdampfdestillation fällt erfindungsgemäß unter die bevorzugten Extraktionsverfahren.

Bevorzugte Pflanzenextrakte sind die Handelsprodukte Extrapone® Puearia Root (INCI-Bezeichnung: Butylene Glycol, Aqua (Water), Pueraria Lobata, Lactic Acid; Fa. Symrise), Extrapone® Sophora Flower (INCI-Bezeichnung: Aqua (Water), Sophora Japonica Flower Extract; Fa. Symrise).

Die erfindungsgemäße weikstoffkombination findet bevorzugt im Rahmen einer oxidativen Haarbehandlung Anwendung. Unter einer oxidativen Haarbehandlung wird erfindungsgemäß die Einwirkung eines oxidativen kosmetischen Mittels, enthaltend in einem kosmetischen Träger mindestens ein Oxidationsmittel, auf Haar definiert.

Die Oxidationsmittel im Sinne der Erfindung sind von Luftsauerstoff verschieden und besitzen ein solches Oxidationspotenzial, das es ermöglicht, Disulfidbrücken innerhalb oder zwischen den Proteinen des Haarkeratins zu knüpfen, das natürliche Farbpigment Melanin oxidativ aufzuhellen und/oder ein Oxidationsfarbstoffvorprodukt vom Entwicklertyp zu oxidieren.

Als Oxidationsmittel kommt bevorzugt Wasserstoffperoxyd und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·n $H_2O_2$ (n ist eine positive ganze Zahl größer 0), Harnstoffperoxid und Melaminperoxid in Frage.

Erfindungsgemäß kann das oxidative kosmetische Mittel auch zusammen mit einem Katalysator auf das Haar aufgebracht werden, der die Oxidation des Substrats, wie beispielsweise Oxidationsfarbstoffvorprodukte oder Melanin, aktiviert. Solche Katalysatoren sind z.B. Metallionen, Iodide, Chinone oder bestimmte Enzyme.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung einer Färbung beschleunigt als auch die Farbnuance gezielt beeinflusst werden.

Geeignete Enzyme sind z.B. Peroxidasen, die die Wirkung geringer Mengen an Wasserstoffperoxyd deutlich verstärken können. Weiterhin sind solche Enzyme erfindungsgemäß geeignet, die mit Hilfe von Luftsauerstoff *in situ* geringe Mengen Wasserstoffperoxyd erzeugen und auf diese Weise die Oxidation der Farbstoffvorprodukte biokatalytisch aktivieren. Besonders geeignete Katalysatoren für die Oxidation von Farbstoffvorprodukten sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Die erfindungsgemäße weikstoffkombination wird bevorzugt unmittelbar vor oder während einer oxidativen Haarbehandlung in Form eines Mittels, enthaltend in einem kosmetischen Träger eine Pflanzchexhahtkombination aus

- Extrakt aus des Warzel von Puea ria Lobata und
- Extrakt aus der Blüte und/oder aus den Knorpen des Sophora japonica L. mit α Liponsäure in Form des freien Säure und/oder desr Salzform verwendet.

Als unmittelbar vor der oxidativen Haarbehandlung versteht sich im Sinne der Erfindung eine Anwendung, an die sich direkt die oxidative Haarbehandlung anschließt, wobei das die Pflanzenextraktkombination enthaltende Mittel zuvor vom Haar gespült oder bevorzugt auf dem Haar belassen wurde und das Haar bevorzugt noch naß ist.

Es hat sich gezeigt, dass die Wirkung der erfindungsgemäßen Pflanzenextraktkombination synergistisch gesteigert werden kann, wenn sie gemeinsam mit α-Liponsäure in Form der freien Säure und/oder der Salzform, verwendet wird.

Die α-Liponsäure kann erfindungsgemäß als freie Säure, aber auch in Gestalt der jeweiligen Salze eingesetzt werden. Die erfindungsgemäß verwendete α-Liponsäure gehorcht somit der Formel (Lip-a)

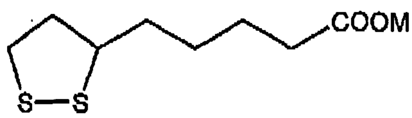

(Lip-a)

worin M ein Wasserstoffatom oder ein Äquivalent eines ein- oder mehrwertigen Kations bedeutet. Wenn die Verbindungen der Formeln (Lip-a) als Säure vorliegen, bedeutet der Rest M ein Wasserstoffatom. Das Fragment -COOM der Formel (Lip-a) bildet In diesem Fall eine Carboxylgruppe. Wenn die Verbindungen der Formeln (Lip-a) als Salz vorliegen, steht M für ein Äquivalent eines ein- oder mehrwertigen Kations. Das ein oder mehrwertige Kation $M^{z+}$ mit einer Ladungszahl z von eins oder höher dient lediglich aus Gründen der Elektraneutralität zur Kompensation der einfach negativen Ladung des bei Salzbildung vorliegenden Carboxylatfragments $-COO^-$ in Formel (Lip-a) bzw. Das dafür zu verwendende Äquivalent des entsprechenden Kations beträgt 1/z. Das Fragment -COOM der Formeln (Lip-a) steht Im Fall der Salzbildung für die Gruppe: $-COO^-$ 1/z $(M^{z+})$.

Als ein- oder mehrwertige Kationen $M^{2+}$ kommen prinzipiell alle physiologisch verträglichen Kationen in Frage. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (Lip-a) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol, M steht in Formel (Lip-a) bevorzugt für ein Wasserstoffatom, ein Ammoniumlon, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kalium, ½ Kalziumion, ½ Magnesiumion oder ½ Zinkion.

Die erfindungsgemäß verwendete $\alpha$-Liponsäure bzw. deren reduzierte Form trägt ein chirales Zentrum am Kohlenstoffatom der sechsten Position des Kohlenstoffgerüstes. Für die erfindungsgemäße Verwendung ist die Stereochemie der $\alpha$-Liponsäure unwesentlich. Folglich können die enantiomerenreinen Formen als auch beliebige Mischungen der Enantiomeren (wie Insbesondere Racemate) erfindungsgemäß verwendet werden.

Die erfindungsgemäß Weihstoffkombination wird bevorzugt in einem kosmetischen Träger verwendet. Als kosmetische Träger eignen sich erfindungsgemäß besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es Ist aber auch denkbar, die Inhaltsstoffe in eine pulverförmige oder auch tablettenförmige Formulierung zu Integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines $C_1$-$C_4$-Alkohols, Insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bei einer gleichzeitigen Anwendung des oxidativen kosmetischen Mittels und einem kosmetischen Mittel, enthaltend die erfindungsgemäße Weihstoffkombination, können beide Mittel, gegebenenfalls erst unmittelbar vor der Anwendung, miteinander gemischt werden, oder nacheinander ohne Zwischenspüischritt auf das Haar aufgebracht werden. In einer bevorzugten Ausführungsform enthält das erfindungsgemäß verwendete oxidative kosmetische Mittel daher zusätzlich die erfindungsgemäße Weihstoffkombination.

Ein zweiter Gegenstand der Erfindung sind kosmetische Mittel, enthaltend in einem kosmetischen Träger eine Pflanzenextraktkombination aus

- Extrakt aus des Wuszel von Puearia Lotata und
- Extrakt aus des Blüte und/oder den Knospen des Sophora japonica L. und $\alpha$-Liponsäure in Form des freien Säure und/oder des Salzform.

Bevorzugte kosmetische Träger sind die des ersten Erfindungsgegenstandes.

Entsprechend bevorzugte Oxidationsmittel wurden ebenfalls im Rahmen des ersten Erfindungsgegenstandes erwähnt. Das Oxidationsmittel ist bevorzugt In einer Menge von 1,0 bis 10 Gew.-%, insbesondere von 3,0 bis 10,0 Gew.-%, jeweils bezogen auf das Gewicht des anwendungsbereiten Mittels, in dem oxidativen kosmetischen Mittel enthalten.

Die erfindungsgemäßen kosmetischen Mittel enthalten jedes der miteinander kombinierten Pflanzenextrakte bevorzugt in einer Menge von 0,01 bis 5.0 Gew.-%, besonders bevorzugt von 0,1 bis 1,5 Gew.-%, ganz besonders bevorzugt von 0,05 bis 0,5 Gew.-%, jeweils bezogen auf das Gewicht des anwendungsbereiten Mittels.

Die erfindungsgemäßen Mittel enthalten zwingend zur synergistischen Steigerung der Wirkung zusätzlich $\alpha$-Liponsäure in Form der freien Säure und/oder der Salzform. Die $\alpha$-Liponsäure in Form der freien Säure und/oder der Salzform ist bevorzugt in den erfindungsgemäßen Mitteln in einer Menge von 0,001 bis 5,0 Gew.-%, besonders bevorzugt von 0,01 bis 2,0 Gew.-%, ganz besonders bevorzugt von 0,1 bis 1 Gew.-%, jeweils bezogen auf das Gewicht des anwendungsbereiten Mittels, enthalten. Desweiteren wird auf das im ersten Erfindungsgegenstand zur Ausführungsform

mit α-Liponsäure Gesagte verwiesen (*vide supra*). Das nachstehend Gesagte gilt selbstverständlich für die Ausführungsform des erfindungsgemäßen Mittels mit α-Liponsäure.

Es ist erfindungsgemäß bevorzugt, das erfindungsgemäße Mittel im Rahmen einer Farbveränderung der Haare, zu verwenden. Zu diesem Zweck enthalten die kosmetischen Mittel zusätzlich mindestens eine farbverändernde Komponente.

Die Farbverändernde Komponente wird wiederum bevorzugt ausgewählt

(a) aus mindestens einem Oxidationsfarbstoffvorprodukt vom Typ der Entwicklerkomponenten und gegebenenfalls zusätzlich mindestens einer Kupplerkomponente
und/oder
(b) aus Oxofarbstoffvorprodukten
und/oder
(c) aus mindestens einem direktziehenden Farbstoff
und/oder
(d) aus mindestens einer Vorstufe naturanaloger Farbstoffe
und/oder
(e) aus mindestens einem Bleichverstärker.

Als Entwicklerkompenenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-([3-[(2-Hydroxyethyl)amino]-4-methoxy-6-methylphenyl}amino) ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5,Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morphollnderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,6-Dimethylresorcin und 2,6-Dihydroxy-3,4-dimethylpyridin und die physiologisch verträglichen Salzen der vorgenannten Verbindungen.

Die erfindungsgemäßen, kosmetischen Mittel enthalten die Entwicklerkomponenten bevorzugt in einer Menge von 0,005 bis 10 Gew.-%, vorzugsweise von 0,1 bis 5 Gew.-%, jeweils bezogen auf das gesamte Mittel.

Die erfindungsgemäßen, kosmetischen Mittel enthalten die Kupplerkomponenten bevorzugt in einer Menge von 0,005 bis 10 Gew.-%, vorzugsweise von 0,1 bis 5 Gew.-%, jeweils bezogen auf das gesamte Mittel.

Das erfindungsgemäße Mittel kann als farbverändernde Komponente in Form der Oxofarbstoffvorprodukte mindestens eine Kombination, aus mindestens einer Verbindung der Komponente

1 Verbindungen, die eine reaktive Carbonylgruppe enthalten mit mindestens einer Verbindung der Komponente
2 Verbindungen, ausgewählt aus (a) CH-aciden Verbindungen, (b) Verbindungen mit primärer oder sekundarer Aminogruppe oder Hydroxygruppe, ausgewählt aus primären oder sekundären aromatischen Aminen, stickstoffhaltigen heterozyklischen Verbindungen und aromatischen Hydroxyverbindungen

enthalten.

Erfindungsgemäße Verbindungen mit einer reaktiven Carbonylgruppe (im Folgenden auch reaktive Carbonylverbindungen oder Komponente 1 genannt) besitzen mindestens eine Carbonylgruppe als reaktive Gruppe, welche mit den Verbindungen der Komponente 2 unter Ausbildung einer beide Komponenten verknüpfenden chemischen Bindung reagiert. Ferner sind erfindungsgemäβ auch solche Verbindungen als Komponente 1 umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente 2 stets vorhanden ist. Diese Derivate sind bevorzugt Kondensationsverbindungen von reaktiven Carbonylverbindungen mit

a) Aminen und deren Derivate unter Bildung von Iminen oder Oximen als Kondensationsverbindung
b) Alkoholen unter Bildung von Acetalen oder Ketalen als Kondensationsverbindung
c) Wasser unter Bildung von Hydraten als Kondensationsverbindung von Aldehyden.

Die Komponente 1 wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacelophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxybenzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxybenzaldehyd, 4-Hydroxy-2,5-dimethoxy-benzaldehyd, 4-Hydroxy-2,6-dimethoxy-benzaldehyd, 4-Hydroxy-2-methylbenzaldehyd, 4-Hydroxy-3-methyl-benzaldahyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dlmethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydraxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-3-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,6-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'- Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylldenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclyhexanon, 2-(4'-Dimethylaminobenzy-liden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 3-Benzyliden-5,5-dimethyl-1,3-cyclohexandion, 2-(4'-Hydroxybenzyliden)-5,5-dimethyl-1,3-cyclohexandion, 2-(4'-Hydroxy-3-meth-oxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl) penta-2,4-dienal, 5-(4-Diethy(aminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitroplperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinotinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-Imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethyl-benzothiazolium-Salze, bevorzugt mit Benzolsulfonat, p-Toluolsulfonat, Methansulfonat, Perchlorat, Sulfat, Chlorid, Bromid, Iodid, Tetrachlorozinkat,

Methylsulfat, Trifluormethansulfonat oder Tetrafluoroborat als Gegenion, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 6-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von elektronenziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente 2 sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-Indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphthol[,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-indoxylacetat, 2-Cumaranon, 5-Hydroxy-2-cumaranon, 5-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumIodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Bevorzugte primäre oder sekundäre aromatische Amine der Komponente 2 sind ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxy-ethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydro-xyethylamino) phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-amlnophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-di-methoxybenzol, 3,5-Diamino-2-methoxy-toluol, 2-Aminobenzoesäure, 3-Aminobenzoesäure, 4-Aminobenzoesaure, 2-Aminophenylessigsäure, 3-Aminophenylessigsäure, 4-Aminophenylessigsäure, 2,3-Diaminobenzoesäure, 2,4-Diaminobenzoesäure, 2,6-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl) amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amlno-2-methyl-6-nitrobenzol, 4-Nitro-1,3-phehylendiamin, 2-Nitro-4-amino-1-(2-hy-droxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel I dargestellt sind

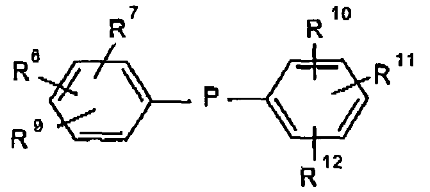

(I)

in der

- $R^7$ für eine Hydroxy- oder eine Aminogruppe, die durch $C_{1-4}$-Alkyl-, $C_{1-4}$-Hydroxyalkyl-, $C_{1-4}$-Alkoxy- oder $C_{1-4}$-Alkoxy-$C_{1-4}$-alkylgruppen substituiert sein kann, steht,
- $R^8$, $R^9$, $R^{10}$, $R^{11}$ und $R^{12}$ unabhängig voneinander für ein Wasserstoffatom, eine Hydroxy- oder eine Aminogruppe, die durch $C_1$-$C_4$-Alkyl-, $C_1$-$C_4$-Hydroxyalkyl, $G_1$-$C_4$-Alkoxy-, $C_1$-$C_4$-Aminoalkyl- oder $C_1$-$C_4$-Alkoxy-$C_1$-$C_4$-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfoxy-, Sulfonyl-oder iminogruppe, ein Sauerstoff- oder Schwefelatom, oder eine Gruppe mit der Formel II

$$-Q'-(CH_2-Q-CH_2-Q'')_o- \qquad (II)$$

in der

- Q eine direkte Bindung, eine $CH_2$- oder CHOH-Gruppe bedeutet,
- Q' und Q" unabhängig voneinander für ein Sauerstoffatom, eine $NR^{13}$-Gruppe, worin $R^{13}$ ein Wasserstoffatom, eine $C_{1-4}$-Alkyl- oder eine Hydroxy-$C_{1-4}$-alkylgruppe, wobei auch beide Gruppen zusammen mit dem Restmolekül einen 5-, 6- oder 7-Ring bilden können, bedeutet, die Gruppe O-$(CH_2)_p$-NH oder NH-$(CH_2)_p$-O, worin p und p' 2 oder 3 sind, stehen und
- o eine Zahl von 1 bis 4 bedeutet,

wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodlphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4.4'-Tetraaminodiphenyl, 3,3,4.4'-Tetraarnino-benzophenon, 1,3-Bis-(2,4-diaminophenoxy)-propan, 1,8-Bis-(2,5-diaminophenoxy)-3,6-dioxaoctan, 1,3-Bis-(4-amino-phenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-aminopyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-dlaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminachinolin, 8-Aminochinolin, 4-Aminochlnaldin, 2-Aminonicotinsäure, 6-Aminonicotinsäure, 5-Aminoisochinolin, 5-Aminoindazol, 6-Aminoindazol, 5-Aminobenzimidazol, 1-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminolndol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Die Verbindungen der Komponente 1 und die Verbindungen der Komponente 2 werden vorzugsweise in den kosmetischen Mitteln jeweils in einer Menge von 0,03 bis 65 mmol, insbesondere von 1 bis 40 mmol, bezogen auf 100 g des gesamten Nuanciermittels, verwendet. Das molare Verhältnis von der Verbindung der Komponente 1 und der Verbindung der Komponente 2 kann im Bereich von 0,5 bis 2,0 liegen, wobei vorzugsweise äquimolare Mengen eingesetzt werden. Das anwendungsbereite Mittel wird bei getrennter Lagerung der Komponenten 1 und 2 unmittelbar vor der Anwendung durch Mischen hergestellt.

Als Vorstufen naturanaloger Farbstoffe werden bevorzugt als Oxidationsfarbstoffvorprodukt vom Eritwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIIa),

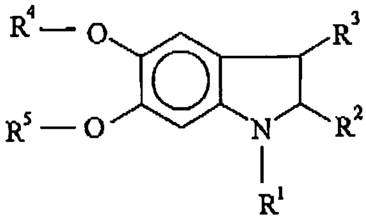

(IIIa)

in der unabhängig voneinander

- $R^1$ steht für Wasserstoff, eine $C_1$-$C_4$-Alkylgruppe oder eine $C_1$-$C_4$-Hydroxy-alkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe.
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^6$ steht für eine der unter $R^4$ genannten Gruppen,

sowie physiologisch verträgliche Salze dieser Verbindungen mit einer organischen oder anorganischen Säure.

Besonders bevorzugte Derivate des indolins sind das 5,6-Dihydroxyindolin, N-Methyl-5,6-dihydroxyindolin, N-Ethyl-5,6-dihydroxyindolin, N-Propyl-6,6-dihydroxyindolin, N-Butyl-5,6-dihydroxylndolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geelgnet sind weiterhin Derivate des 5,6-Dihydroxyindols der Formel (IIIb),

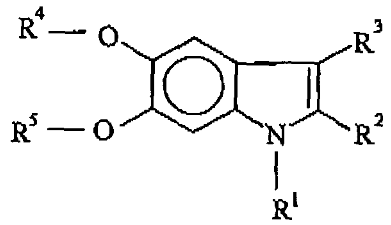

(IIIb)

in der unabhängig voneinander

- $R^1$ steht für Wasserstoff, eine $C_1$-$C_4$-Alkylgruppe oder eine $C_1$-$C_4$-Hydroxyalkylgruppe,
- $R^2$ steht für Wasserstoff oder eine -COOH-Gruppe, wobei die -COOH-Gruppe auch als Salz mit einem physiologisch verträglichen Kation vorliegen kann,
- $R^3$ steht für Wasserstoff oder eine $C_1$-$C_4$-Alkylgruppe,
- $R^4$ steht für Wasserstoff, eine $C_1$-$C_4$-Alkylgruppe oder eine Gruppe -CO-$R^6$, in der $R^6$ steht für eine $C_1$-$C_4$-Alkylgruppe, und
- $R^5$ steht für eine der unter $R^4$ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Bevorzugte direktziehende Farbstoffe, die In den kosmetischen Mitteln als farbverändernde Komponente Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphe-nylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die kosmetischen Mittel einen kationischen direktzlehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

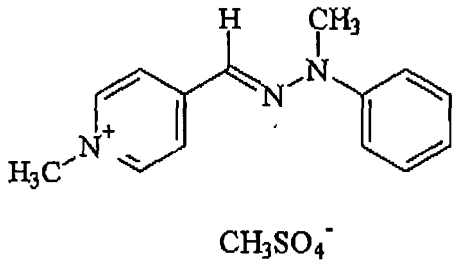

(DZ1)

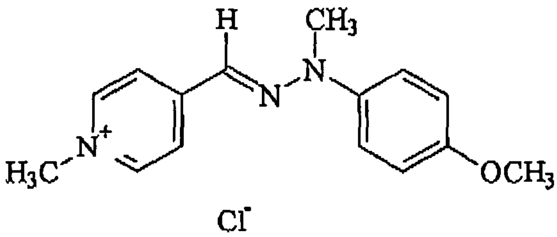

(DZ2)

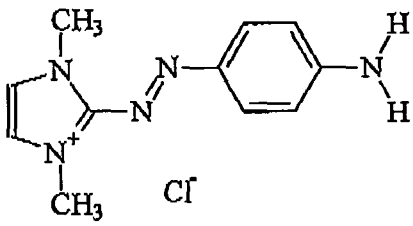

(DZ3)

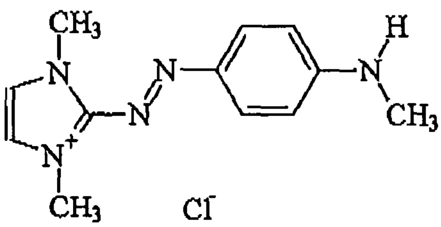

(DZ4)

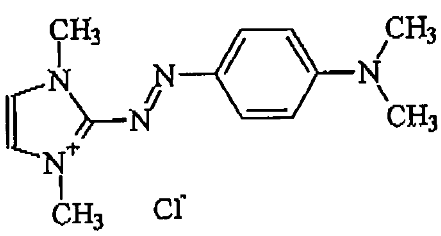

(DZ5)

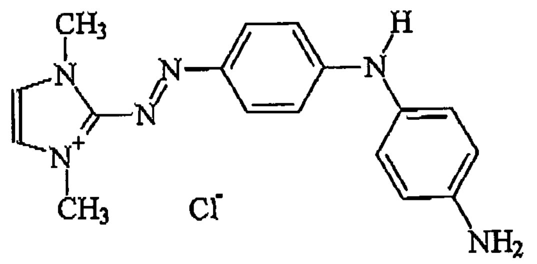

(DZ6)

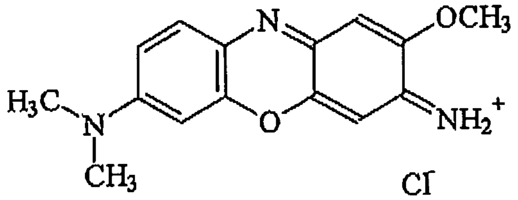

(DZ7)

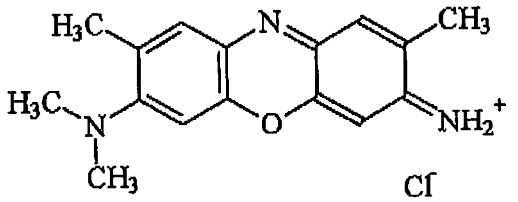

(DZ8)

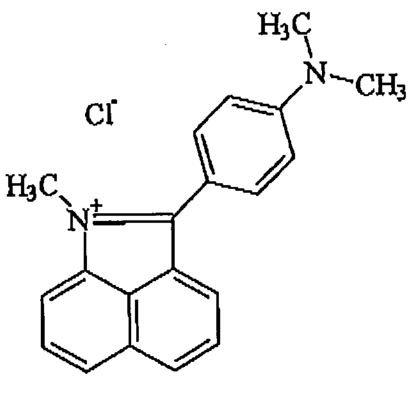

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die kosmetischen Mittel enthalten die direktziehenden Farbstoffe bevorzugt in einer Menge von 0,01 bis 20 Gew.-%, bezogen auf das anwendungsbereite Mittel.

Weiterhin können die erfindungsgemäßen kosmetischen Mittel auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxldationsfarbstoffvorprodukte oder die direktziehenden Farbstoffe jeweils einheitliche Verbindungen darstellen. Vielmehr können in den kosmetischen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen

werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh, Schrader, Grundlagen und Rezepturen der Kosmetika, 2. Auflage, Hüthig Buch Verlag, Heidelberg, 1989, verwiesen.

Das eigentliche oxidative Färbemittel wird bei getrennter Lagerung der Farbstoffvorprodukte und des Oxidationsmittels unmittelbar vor der Anwendung durch Mischen hergestellt. In einer bevorzugten Ausführungsform wird daher das kosmetische Mittel vor der Applikation aus einer Zusammensetzung, enthaltend in einem kosmetischen Träger mindestens eine farbverändernde Komponente, und einer weiteren Zusammensetzung, enthaltend in einem kosmetischen Träger mindestens ein Oxidationsmittel, gemischt.

Bei einer Anwendung von Öxidationsmittein wird die anwendungsfertige Zubereitung zweckmäßlgerweise unmittelbar vor der Anwendung durch Mischung einer Zusammensetzung, enthaltend das Oxidationsmittel mit der Zusammensetzung, enthaltend die farbverändernden Komponenten, hergestellt. Das dabei entstehende gebrauchsfertige Haarpräparat sollte bevorzugt einen pH-Wert im Bereich von 6 bis 12, insbesondere von pH 7,5 bis 10, aufweisen.

Für eine Farbveränderung mittels Aufhellung bzw. Bleichung der Haare wird bevorzugt in den erfindungsgemäßen kosmetischen Mitteln neben den Oxidationsmitteln zusätzlich mindestens ein Bleichverstärker eingesetzt.

Bleichverstärker werden bevorzugt in Blondiermitteln zur Steigerung der Blondierwirkung des Oxidationsmittels, insbesondere des Wasserstoffperoxyds, eingesetzt.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, Insbesondere 1,5-Diacetyl-2,4-dioxohexahydro-1,3,5-triazin (DADHT), acylierte Glykolurile, insbesondere Tetraacetylglykolutil (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acyllerte mehrwertige Alkohole, insbesondere Triacetin, Ethylenglykoldiacetat und 2,5-Diacetoxy-2,5-dihydrofuran.

Als Bleichverstärker können erfindungsgemäß bevorzugt Carbonatsalze, bzw. Hydrogencarbonatsalze eingesetzt werden. Diese sind bevorzugt ausgewählt aus der Gruppe der Ammonium-, Alkali- (insbesondere Natrium- und Kalium-), sowie Erdalkali- (insbesondere Calcium-), -carbonatsalze bzw. -hydrogencarbonatsalze. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (IV),

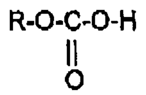

(IV)

in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (IV) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine $C_{1-6}$-Alkylgruppe. Beispiele für erfindungsgemäße $C_1$-$C_6$-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (IV) ausgewählt ist aus Methyl-, Ethyl-, n-Propyl-, iso-Propyl-, n-Butyl-, iso-Butyl-, tert-Butyl- sowie Hydroxymethyl- und Hydroxyethyl-Resten.

Alternativ zum Kohlensäuremonoester oder in Verbindung mit ihm können in den wasserfreien Zusammensetzungen Kohlensäuremonoamide als Bleichverstärker eingesetzt werden. Hier ist es erfindungsgemäß bevorzugt, mindestens ein Kohlensäuremonoamid der Formel (V) zu verwenden,

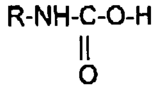

(V)

in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (V) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine $C_{1-6}$-Alkylgruppe. Beispiele für erfindungsgemäße $C_1$-$C_6$-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (V) sind dadurch gekennzeichnet, daß der Rest R in Formel (V) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet. Bevorzugt werden Silylcarbonate gemäß Formel (VI) eingesetzt,

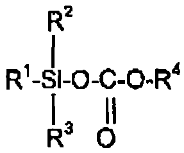

(VI)

in der die Reste $R^1$, $R^2$ und $R^3$ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und der Rest $R^4$ für eine chemische Bindung zum Si-Atom oder zu einem der Reste $R^1$, $R^2$ oder $R^3$, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste $R^1$, $R^2$ und $R^3$ in der oben genannten Formel (VI) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße wasserfreie Zusammensetzungen dadurch gekennzeichnet sind, daß die Reste $R^1$, $R^2$ und $R^3$ in Formel (VI) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste $R^4$ in der oben genannten Formel (VI) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als Bleichverstärker kann mindestens ein Silylcarbamat der Formel (VII) in der erfindungsgemäßen wasserfreien Zusammensetzung enthalten sein,

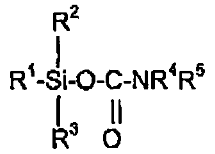

(VII)

wobei die Reste $R^1$, $R^2$ und $R^3$ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trlmethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und die Rest $R^4$ und $R^6$ unabhängig voneinander für eine chemische Bindung zum Si-Atom oder zu einem der Reste $R^1$, $R^2$ oder $R^3$, ein Wasserstoffatom, einen gesättigten oder ungesättigten, geradkettigen, verzweigten, oder cyclischen, substituierten oder unsubstituierten Kohlenwasserstoffrest oder für eine substituierte

oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste $R^1$, $R^2$ und $R^3$ in der oben genannten Formel (VII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste $R^1$, $R^2$ und $R^3$ in Formel (VII) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste $R^4$ und $R^5$ in der oben genannten Formel (VII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Bleichverstärker sind bevorzugt Peroxoverbindungen. Unter die erfindungsgemäß bleichverstärkenden Peroxoverbindungen fallen keine Anlagerungsprodukte von Wasserstoffperoxyd an andere Komponenten und auch nicht Wasserstoffperoxyd selbst. Die Auswahl der Peroxoverbindungen unterliegt darüber hinaus keinen Beschränkungen. Bevorzugte Peroxoverbindungen sind Peroxidisulfatsalze, Persulfatsalze, (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Bleichverstärker sind in den erfindungsgemäßen, kosmetischen Mitteln bevorzugt in Mengen von 5-30 Gew.-%, insbesondere in Mengen von 8-20 Gew.-%, enthalten.

Die kosmetischen Mittel der Erfindung enthalten, wenn sie als Bleichmittel fungieren, als bevorzugtes Alkalisierungsmittel mindestens eine Verbindung, ausgewählt aus Ammonlum-, Alkalimetall- und Erdalkalimetallhydroxiden, -carbonaten, -hydrogencarbonaten, -hydroxycarbonaten, -metasilikaten und -carbamiden, sowie Alkaliphosphaten.

Die in dem erfindungsgemäßen Verfahren eingesetzten kosmetischen Mittel können weiterhin alle für solche Zubereitungen bekannten Wirk-, Zusatz- und Hilfsstoffe enthalten:

Das Nachwaschen mit einem Shampoo entfällt, wenn ein stark tensidhaltiger Träger verwendet wurde.

In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den kosmetischen Mitteln alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,

- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen.
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und x = 0 oder 1 bis 12 ist,
- anionische Alkyloligogiykoside bzw. anionische Alkenyloligoglykosid-Derivate, ausgewählt aus Alkyl- und/oder Alkenyl-Oligoglykosidcarboxylaten, -sulfaten, -phosphaten und/oder - isethiohaten, die sich von Alkyl- und/oder Alkenyloligoglykosiden der allgemeinen Formel (VIII) ableiten,

$$R-O-(G)_p \qquad (VIII)$$

mit der Bedeutung
R $C_{6-22}$-Alkyl oder $C_{6-22}$-Alkenyl,
G Glykosideinheit, die sich von einem Zucker mit 5 oder 6 Kohlenstoffatomen ableitet,
p Zahl von 1 bis 10,
insbesondere das Laurylglucosidcarboxylat, wie es als Plantapon® LGC von Cognis Deutschland erhältlich ist,

- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Nichtionogene Tenside enthalten als hydrophile Gruppe z. B, eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$Fettsauremono- und -diester von Anlagerungsprodukten von 1 bis 30 Mol Ethylenoxid an Glycerin,
- $C_6$-$C_{22}$-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel $R^1O-(Z)_x$. Diese Verbindungen sind durch die folgenden Parameter gekennzeichnet.

Der Alkylrest $R^1$ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Oetyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest $R^1$ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen $R^1$

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder

- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer $C_6$-$C_{18}$-Alkyl- oder Acylgruppe im Molekül mindestens eine freie Aminogruppe und mindestens eine - COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminoproplonat, das Kokosacylaminoethylaminopropionat und das $C_{12-18}$-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis (2-Palmitoyloxyethyl)dimethylammoniumchlorid, sowie Dehyquart® F-75 und Dehyquar® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Sillkonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric),

SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handeisprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

In einer weiteren Ausführungsform enthalten die erfindungsgemäßen Mittel zusätzlich mindestens ein kationisches und/oder mindestens ein amphoteres Polymer.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (P1),

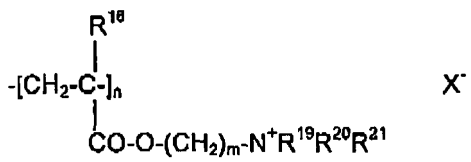

(P1)

in der $R^{18}$ = -H oder $-CH_3$ ist, $R^{19}$, $R^{20}$ und $R^{21}$ unabhängig voneinander ausgewählt sind aus $C_{1-4}$-Alkyl-, -Alkenyl- oder-Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (P1) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

- $R^{16}$ steht für eine Methylgruppe
- $R^{19}$, $R^{20}$ und $R^{21}$ stehen für Methylgruppen
- m hat den Wert 2.

Als physiologisch verträgliches Gegenionen $X^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INGI-Bezeichnung: Mineral Oil) und Tridecyl-polyoxypropylen-polyoxyethylen-ether *(INCI-Bezeichnung: PPG-1-Trideceth-6)) und Salcare® SC 96 (ca. 50 % Polymeranteil,

weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (P1) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren Ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- katlonlserter Honig, beispielsweise das Handelsprodukt Honeyquat® 50,
- kationische Guar-Derivate, wie insbesondere die unter den Handelsnamen Casmedla®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und methacrylats, wie beispielsweise mit Dimethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,

sowie die unter den Bezeichnungen

- Polyquaternium 2 (z.B. Mirapol® A-15 der Firma Rhodia),
- Polyquaternium 17,
- Polyquaternium 18 und
- Polyquaternium 27 bekannten Polymeren mit quartären Stickstoffatomen in der Polymerhauptkette,

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von $5 \cdot 10^5$ bis $5 \cdot 10^6$ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-6-carbonsäure, Benzoesäure oder Salicylsäure. Bevorzugt werden niedermolekulare Hydroxycarbonsäuren wie z.B. Glycolsäure oder Milchsäure verwendet.

Unter dem Begriff amphotere Polymere werden solche Polymere verstanden,

- die im Molekül sowohl freie Aminogruppen als auch freie -COOH- oder $SO_3H$-Gruppen enthalten und zur Ausbildung innerer Salze befähigt sind,
- zwitterionische Polymere, die im Molekül quartäre Ammoniumgruppen und -COO'-Gruppen oder $-SO_3^-$-Gruppen enthalten, sowie
- Polymere, die -COOH-Gruppen oder $SO_3H$-Gruppen und quartäre Ammoniumgruppen enthalten.

Die in der Aufzählung genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 2B 17 369 genannten Verbindungen.

Erfindungsgemäß bevorzugte amphotere und/oder kationische Polymere sind solche Polymerisate, in denen sich eine kationische Gruppe ableitet von mindestens einem der folgenden Monomere:

- Monomeren mit quartären Ammoniumgruppen der allgemeinen Formel (M1),

$$R^1\text{-CH=CR}^2\text{-CO-Z-}(C_nH_{2n})\text{-N}^{(+)}R^3R^4R^5\ A^{(-)} \qquad (M1)$$

  in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist,

- Monomeren mit quartären Ammoniumgruppen der allgemeinen Formel (M2),

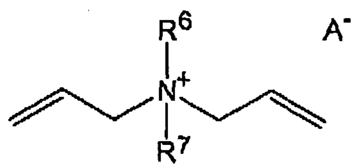

(M2)

  worin $R^6$ und $R^7$ unabhängig voneinander stehen für eine ($C_1$ bis $C_4$)-Alkylgruppe, insbesondere für eine Methylgruppe und
  $A^-$ das Anion einer organischen oder anorganischen Säure ist.

Wenn sich eine kationische Gruppe der amphoteren bzw. kationischen Polymerisate vom Monomer der Formel (M1) ableitet, stehen die Reste $R^3$, $R^4$ und $R^5$ in Formel (M1) bevorzugt für Methylgruppen, Z ist bevorzugt eine NH-Gruppe und $A^{(-)}$ bedeutet bevorzugt ein Halogenid-, Methoxysulfat- oder Ethoxysulfat-Ion. Besonders bevorzugt Ist es in diesem Falle Acrylamidopropyl-trimethyl-ammoniumchlorid als Monomer (M1) zu verwenden.

In Formel (M2) steht $A^-$ bevorzugt für ein Halogenidion, insbesondere für Chlorid oder Bromid.

Bevorzugte erfindungsgemäße amphotere Polymere sind Polymere, deren anionische Gruppe sich von mindestens einem Monomeren der Formel (M3) ableitet

- monomeren Carbonsäuren der allgemeinen Formel (M3) bzw. deren Salze mit einer organischen oder anorganischen Säure,

$$R^8\text{-CH=CR}^9\text{-COOH} \qquad (M3)$$

in denen $R^8$ und $R^9$ unabhängig voneinander Wasserstoff oder Methylgruppen sind.

Als Monomeres (M3) wird für die erfindungsgemäß bevorzugten amphoteren Polymerisate Acrylsäure verwendet.

Besonders bevorzugte amphotere Polymere sind Copolymere, aus mindestens einem Monomer (M1) bzw. (M2) mit dem Momomer (M3), insbesondere Copolymere aus den Monomeren (M2) und (M3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.

Darüber hinaus können die erfindungsgemäßen amphoteren Polymere neben einem Monomer (M1) oder M (2) und einem Monomer (M3) zusätzlich ein Monomer (M4)

- monomere Carbonsäureamide der allgemeinen Formel (M4),

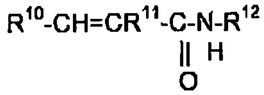 (M4)

in denen $R^{10}$ und $R^{11}$ unabhängig voneinander Wasserstoff oder Methylgruppen sind und $R^{12}$ für ein Wasserstoffatom oder eine ($C_1$- bis $C_8$)-Alkylgruppe steht, enthalten.

Erfindungsgemäß ganz besonders bevorzugt verwendete amphotere Polymere auf Basis eines Comonomers (M4) sind Terpolymere aus Diallyldimethylammoniumchlorid, Acrylamid und

Acrylsäure. Diese Copolymerisate werden unter der INCI-Bezeichnung Polyquatemium-39 unter anderem mit dem Handelsnamen Merquat® Plus 3330 (Nalco) vertrieben.

Besonders bevorzugt eingesetzte amphotere Polymere sind solche Polymerisate, die sich im wesentlichen zusammensetzen aus

(i) Monomeren mit quartären Ammoniumgruppen der allgemeinen Formel (M1),

$R^1$-CH=C$R^2$-CO-Z-($C_nH_{2n}$)-$N^{(+)}R^2R^3R^4$ $A^{(-)}$ in der $R^1$ und $R^2$ (M1)

unabhängig voneinander stehen für Wasserstoff oder eine Methylgruppe und $R^3$, $R^4$ und $R^6$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist
und
(ii) monomeren Carbonsäuren der allgemeinen Formel (M3),

$R^5$-CH=C$R^9$-COOH (M3)

in denen $R^0$ und $R^9$ unabhängig voneinander Wasserstoff oder Methylgruppen sind.

Bezüglich der Einzelheiten der Herstellung dieser besonders bevorzugten Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (i) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethylammonlumchlorid ist ein besonders bevorzugtes Monomeres (i). Als Monomeres (ii) für die genannten Polymerisate wird bevorzugt Acrylsäure verwendet.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Bevorzugte erfindungsgemäße Mittel enthalten in einem kosmetischen Träger neben der Kombination aus (a) mindestens einem Oxidationsmittel (bevorzugt Wasserstoffperoxid) und (b) $\alpha$-erfindungsgemäßen Pflanzenextraktkombination, zusätzlich eine der folgenden Wirkstoffkombinationen (i) bis (xii):

(i) mindestens eine farbverändernde Komponente und mindestens ein kationisches Polymer,
(ii) mindestens eine farbverändernde Komponente und mindestens ein amphoteres Polymer,

(iii) mindestens eine farbverändernde Komponente und mindestens ein kationisches Polymer, abgeleitet von mindestens einem Monomer aus den oben genannten Formeln (M1) und/oder (M2),
(iv) mindestens eine farbverändernde Komponente und mindestens ein amphoteres Copoylmer, das ist im wesentlichen zusammensetzt aus mindestens einem Monomer der Formel (M1) und mindestens einem Monomer der Formel (M3),
(v) mindestens eine Entwicklerkomponente (und gegebenenfalls eine Kupplerkomponente) und mindestens ein kationisches Polymer,
(vi) mindestens eine Entwicklerkomponente (und gegebenenfalls eine Kupplerkomponente) und mindestens ein amphoteres Polymer,
(vii) mindestens eine Entwicklerkomponente (und gegebenenfalls eine Kupplerkomponente) und mindestens ein kationisches Polymer, abgeleitet von mindestens einem Monomer aus den oben genannten Formeln (M1) und/oder (M2),
(viii) mindestens eine Entwicklerkomponente (und gegebenenfalls eine Kupplerkomponente) und mindestens ein amphoteres Copoylmer, das ist im wesentlichen zusammensetzt aus mindestens einem Monomer der Formel (M1) und mindestens einem Monomer der Formel (M3).
(ix) mindestens einem Bleichverstärker und mindestens ein kationisches Polymer,
(x) mindestens einem Bleichverstärker und mindestens ein amphoteres Polymer,
(xi) mindestens einem Bleichverstärker und mindestens ein kationisches Polymer, abgeleitet von mindestens einem Monomer aus den oben genannten Formeln (M1) und/oder (M2),
(xii) mindestens einem Bleichverstärker und mindestens ein amphoteres Copoylmer, das ist im wesentlichen zusammensetzt aus mindestens einem Monomer der Formel (M1) und mindestens einem Monomer der Formel (M3).

In einer weiteren bevorzugten Ausführungsform der Erfindung kann die Wirkung durch Zusatz von mindestens einem Fettstoff weiter optimiert werden. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.
Als Fettsäuren können eingesetzt werden lineare und/oder verzweigte, gesättigte und/oder ungesättigte Fettsäuren mit 6-30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasaure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.
Die Einsatzmenge beträgt dabei 0,1 - 15 Gew.%, bezogen auf das gesamte Mittel. In einer bevorzugten Ausführungsform beträgt die Menge 0,5-10 Gew.%, wobei ganz besonders vorteilhaft Mengen von 1-5 Gew.% sind.
Als Fettalkohole können eingesetzt werden gesättigte, ein- oder mehrfach ungesättigte, verzweigte oder unverzweigte Fettalkohole mit $C_6$ - $C_{30}$-, bevorzugt $C_{10}$ - $C_{22}$- und ganz besonders bevorzugt $C_{12}$ - $C_{22}$- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäuteestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Flullan® käuflich zu erwerben sind, eingesetzt werden. Die Fettalkohole werden in Mengen von 0,1 - 20 Gew.-%, bezogen auf die gesamte Zubereitung, bevorzugt in Mengen von 0,1-10 Gew.-% eingesetzt.
Als natürliche oder synthetische Wachse können erfindungsgemäß eingesetzt werden feste Paraffine oder

Isoparaffine, Carnaubawachse, Bienenwachse, Candelillawachse, Ozokerite, Ceresin, Walrat, Sonnanblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, DI-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, DI-iso-pentylether, Di-3-ethyldecylether, tert-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol®OE) können bevorzugt sein.
- Esteröle. Unter Esterölen sind zu verstehen die Ester von $C_6$ - $C_{30}$ - Fettsäuren mit $C_2$ - $C_{30}$ Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmilat (Cegesoft® 24), Stearinsäure-2-ethylhexylester (Cetiol® 868), Cetyloleat, Glycerintriraprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetilol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adlpat, Di-(2-ethylhexyl)-succinat und Di-Isotriderylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12 oder Cutina® MD.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 6-45 Gew.-%, bezogen auf das gesamte Mittel. Mengen von 10- 35 Gew.-% sind erfindungsgemäß bevorzugt.

Weiterhin hat sich gezeigt, daß die Wirkung gesteigert werden kann, wenn die erfindungsgemäßen Mittel zusätzlich mindestens einen Hydroxycarbonsäureester enthalten. Bevorzugte Hydroxycarbonsäureester sind Vollester der Glycolsäure, Milchsäure, Äpfelsäure, Weinsäure oder Citronensäure. Weitere grundsätzlich geeigneten Hydroxycarbonsäuraester sind Ester der ß-Hydroxypropionsäure, der Tartronsäure, der D-Gluconsäure, Zuckersäure, Schielmsäure oder Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von $C_{12}$-$C_{15}$-Fettalkoholen besonders bevorzugt. Ester dieses Typs sind im Handel erhältlich, z.B. unter dem Warenzeichen Cosmacal® der EniChem, Augusta Industriale. Die Einsatzmenge der Hydroxycarbonsäureester beträgt dabei 0,1 - 15 Gew.% bezogen auf das Mittel, bevorzugt 0,1 - 10 Gew.% und ganz besonders bevorzugt 0,1 - 5 Gew.%.

Bevorzugte erfindungsgemäße Mittel enthalten in einem kosmetischen Träger neben der Kombination aus (a) mindestens einem Oxidationsmittel (bevorzugt Wasserstoffperoxid) und (b) der erfindungsgemäßen Pflanzenextraktkombination, zusätzlich eine der folgenden Wirkstoffkombinationen (i) bis (xii):

(i) mindestens eine farbverändernde Komponente und mindestens einen Fettstoff,
(ii) mindestens eine farbverändernde Komponente und mindestens einen $C_{10}$ bis $C_{22}$-Fettalkohol,
(iii) mindestens eine farbverändernde Komponente und mindestens eine $C_{10}$ bis $C_{22}$-Fettsäure,
(iv) mindestens eine farbverändernde Komponente und mindestens ein natürliches oder synthetisches Wachs,
(v) mindestens eine farbverändernde Komponente und mindestens einen natürlichen oder synthetischen Ölkörper,
(vi) mindestens Entwicklerkomponente (und gegebenenfalls eine Kupplerkomponente) und mindestens einen Fettstoff,
(vii) mindestens eine Entwicklerkomponente (und gegebenenfalls eine Kupplerkomponente) und mindestens einen $C_{10}$ bis $C_{22}$-Fettalkohol,
(viii) mindestens eine Entwicklerkomponente (und gegebenenfalls eine Kupplerkomponente) und mindestens eine $C_{10}$ bis $C_{22}$-Fettsäure,
(ix) mindestens eine Entwicklerkomponente (und gegebenenfalls eine Kupplerkomponente) und mindestens ein natürliches oder synthetisches Wachs,
(x) mindestens eine Entwicklerkomponente (und gegebenenfalls eine Kupplerkomponente) und mindestens einen natürlichen oder synthetischen Ölkörper.
(xi) mindestens einen Bleichverstärker und mindestens einen Fettstoff,
(xii) mindestens einen Bielchverstärker und mindestens einen $C_{10}$ bis $C_{22}$-Fettalkohol,
(xiii) mindestens einen Bleichverstärker und mindestens eine $C_{10}$ bis $C_{22}$-Fettsäure,
(xiv) mindestens einen Bleichverstärker und mindestens ein natürliches oder synthetisches Wachs,
(xv) mindestens einen Bleichverstärker und mindestens einen natürlichen oder synthetischen Ölkörper.

Es ist erfindungsgemäß bevorzugt, dass die Mittel zusätzlich mindestens ein Alkalisierungsmittel enthalten. Die erfindungsgemäß verwendbaren Alkalisierungsmittel werden bevorzugt ausgewählt aus der Gruppe, die gebildet wird, aus Ammoniak, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$-$C_8$-Alkylgrundkörper, der mindestens eine Hydroxylgruppe trägt. Besonders bevorzugte Alkanolamine werden aus der Gruppe ausgewählt, die gebildet wird, aus 2-Aminoethen-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxid, L-Arginin, D-Arginin, DL-Arginin, N-Methylglucamin, Morpholin, Imidazol und Harnstoff.

Bevorzugte erflndungsgemaße Mittel enthalten in einem kosmetischen Träger neben der Kombination aus (a) mindestens einem Oxidationsmittel (bevorzugt Wasserstoffperoxid) und (b) der erfindungsgemäßen Pflanzenextraktkombination, zusätzlich eine der folgenden Wirkstoffkombinationen (i) bis (xii):

(i) mindestens eine farbverändernde Komponente und Ammoniak,
(ii) mindestens eine farbverändernde Komponente und mindestens ein Alkanolamin,
(iii) mindestens eine farbverändernde Komponente und 2-Aminoethan-1-ol,
(iv) mindestens eine farbverändernde Komponente und Ammoniak und 2-Aminoethan-1-ol,
(v) mindestens eine farbverändernde Komponente und 2-Aminoethan-1-ol und L-Arginin,
(vi) mindestens Entwicklerkomponente (und gegebenenfalls eine Kupplerkomponente) und Ammoniak,
(vii) mindestens eine Entwicklerkomponente (und gegebenenfalls eine Kupplerkomponente) und mindestens ein Alkanolamin,
(viii) mindestens eine Entwicklerkomponente (und gegebenenfalls eine Kupplerkomponente) und 2-Aminoethan-1-ol,

(ix) mindestens eine Entwicklerkomponente (und gegebenenfalls eine Kupplerkomponente) und Ammoniak und 2-Aminoethan-1-ol,
(x) mindestens eine Entwicklerkomponente (und gegebenenfalls eine Kupplerkomponente) und 2-Aminoethan-1-ol und L-Arginin,
(xi) mindestens einen Bleichverstärker und Ammoniak,
(xii) mindestens einen Bleichverstärker und mindestens ein Alkanolamin,
(xiii) mindestens einen Bleichverstärker und 2-Aminoethan-1-ol,
(xiv) mindestens einen Bleichverstärker und Ammoniak und 2-Aminoethan-1-ol,
(xv) mindestens einen Bleichverstärker und 2-Aminoethan-1-ol und L-Arginin.

In einer weiteren Ausführungsform sollten die erfindungsgemäßen Mittel zusätzlich mindestens einen UV-Lichtschutzfilter enthalten. Unter UV-Lichtschutzfiltern sind organische Substanzen zu verstehen, die in der Lage sind, ultra-violette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substan-zen sind z.B. zu nennen:

- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino) benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfünsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylldenmethyl)benzolsul-fonsäure und 2-Methyl-5-(2-oxo-3-bornyliden)sulfonsäure und deren Salze.

Als typische UV-A-Filter kommen insbesondere Derivate des Benzoylmethans in Frage, wie beispiels-weise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch In Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Weiterhin ist es bevorzugt, in die erfindungsgemäßen Mittel zusätzlich mindestens ein Pflanzenextrakt einzuarbeiten.

Geeignete Pflanzenextrakte werden durch Extraktion mit organischen Lösemitteln (wie beispielsweise Ethanol, Isopropanol, Diethylether, Benzin, Benzol, Chloroform) oder durch Wasserdampfdestillation gewonnen. Bevorzugte Pflanzenextrakte sind beispielsweise Extrakte von Blüten (Lindenblüten, Kamillen, Lilie, Lavendel, Rosen, Jasmin, Neroli, Ylang-Ylang), Stengeln und Blättern (Cassis, Rosskastanie, Rooibos, Birke, Melisse, Klee, Weinblättern, Geranium, Patchouli, Petitgrain), Früchten (Anis, Cassis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian, Rosmarin), Nadeln und Zweigen

(Fichte, Tanne, Kiefer, Latschen, Sandelholz), Harzen und Balsamen (Galbanum, Elemi, Benzoe, Myrrhe, Olibanum, Opoponax).

Besonders bevorzugt werden die Pflanzenextrakte ausgewählt aus mindestens einem Extrakt aus der Gruppe Hamamelis (Hamamelis virginiana L.), Weinblättern (Vitis vinifera L.), Rosen (Rosa gallica L.), Sandelholz (Pterocarpus Santalinus), Rooibos (Aspalathus linearis), Rosskastanie (Aesculus Hippocastanum L.), Klee (insbesondere Rotklee, Trifolium pratense), Zimt (Cinnamomum zeylanicum nees) und Cassis (insbesondere aus Cassis Blättern, Ribes nigrum L.).

Solche bevorzugt verwendeten Extrakte werden unter den Bezeichnungen Herbasol® von der Firma Cosmetochem oder Extrapon® von der Firma Symrise vermarktet.

Die Pflanzenextrakte sind in dem erfindungsgemäßen Mittel bevorzugt in einer Menge von 0,05 bis 5 Gew.-%, insbesondere 0,1 bis 2 Gew.-%, jeweils bezogen auf das Gewicht des gesamten Mittels, enthalten.

Ferner können die kosmetischen Mittel weitere Wirk-, Hilfs- und Zusatzstoffe enthalten, wie beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylaceta/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, Insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbel-, Rosmarin, Birke, Malve, Wiesenschaumkraut, Quendel, Schafgarbe, Thymian, Melisse, Hauhechel, Huflattich, Elbisch, Meristem, Ginseng und Ingwerwurzel,
- Cholesterin,
- Konsistenzgeber wie Zuckerester, Polyolester oder Polyolalkylether,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente.
- Stabilislerungsmittel für Wasserstoffperoxyd und andere Oxidationsmittel,
- Treibmittel wie Propan-Butan-Gemische, $N_2O$, Dimethylether, $CO_2$ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Für den zweiten Erfindungsgegenstand gilt *mutatis mutandis* das für den ersten Erfindungsgegenstand Gesagte.

Ein dritter Erfindungsgegenstand ist ein Verfahren zur Haarbehandlung, In dem ein ein kosmetisches Mittel, enthaltend in einem kosmetischen Träger die erfindungsgemäße weikstoffkombination aufgetragen und gegenbenfalls nach einer Einwirkungszeit wieder abgespillt wird.

Als bevorzugte Ausführungsform gilt ein Verfahren, in dem ohne Zwischenspülen unmittelbar nach dem Auftragen des kosmetischen Mittels, enthaltend in einem kosmetischen Träger die erfindungsgemäße weikstoffkombination

- ein oxidatives kosmetisches Mittel, enthaltend in einem kosmetischen Träger mindestens ein Oxidationsmittel, aufgetragen und gegebenenfalls nach einer Einwirkungszeit wieder abgespült wird
  und/oder

- das kosmetische Mittel zusätzlich mindestens ein Oxidationsmittel enthält.

Die Einwirkungszeit der weikstoffkombination betägt bevorzugt 1 bis 100 Minuten, besonders bevorzugt 5 bis 50 Minuten.

Es ist wiederum erfindungsgemäß bevorzugt das erfindungsgemäße Verfahren im Rahmen einer oxidativen Haarfärbung, der oxidativen Haarbleiche oder der Fixierung im Rahmen einer Dauerwelle anzuwenden. Dabei ist es bevorzugt, die erfindungsgemäße weikstoffkombination gemeinsam mit dem Oxidationsmittel auf dem Haar anzuwenden.

In einer bevorzugten Ausführungsform des erfindungsgemäßen Verfahrens als oxidatives Haarfärbeverfahren, wird bevorzugt die erfindungsgemäße weikstoffkombination als Bestandteil eines kosmetischen Mittels enthaltend in einem kosmetischen Träger zusätzlich mindestens ein Oxidationsmittel und mindestens ein Oxidationsfarbstoffvorprodukt in einem Schritt angewendet.

Die oxidativen Haarfärbemittel dieser Ausführungsform sind bevorzugt Zweikomponenten Mittel. Die erste Komponente enthält in einem kosmetischen Träger die erfindungsgemäße weikstoffkombination und mindestens ein Oxidationsfarbstoffvorprodukt. Die zweite Komponente enthält in einem kosmetischen Träger mindestens ein Oxidationsmittel. Diese Komponenten werden bevorzugt getrennt voneinander in jeweils einem Kompartiment konfektioniert und gemeinsam in einer Verpackungseinheit (Kit) bereitgestellt. Kurz vor der Anwendung werden beide Komponenten miteinander vermischt.

In der Ausführungsform des erfindungsgemäßen Verfahrens als oxidatives Haarbleichverfahren, wird bevorzugt die erfindungsgemäße weikstoffkombination als Bestandteil eines kosmetischen Mittels enthaltend in einem kosmetischen Träger zusätzlich mindestens ein Oxidationsmittel und mindestens einen Bleichverstärker in einem Schritt angewendet.

Die oxidativen Haarbleichmittel dieser Ausführungsform sind bevorzugt Zwel- oder Dreikomponenten-Mittel. Die erste Komponente enthält in einem kosmetischen Träger die erfindungsgemäße weikstoffkombination und gegebenenfalls mindestens einen Bleichverstärker. Der Bleichverstärker kann auch getrennt von der erfindungsgemäßen weikstoffkombination konfektioniert sein, beispielsweise in Pulverform, als wasserfreie Paste oder wasserfreies Öl. Dadurch wird ein Dreikomponenten-Mittel erhalten. Die letzte Komponente enthält in einem kosmetischen Träger mindestens ein Oxidationsmittel. Alle Komponenten werden bevorzugt getrennt voneinander in jeweils einem Kompartiment konfektioniert und gemeinsam in einer Verpackungseinheit (Kit) bereitgestellt. Kurz vor der Anwendung werden alle Komponenten miteinander gemischt.

Die zuvor beschriebenen Mehrkomponentenmittel zur Durchführung des erfindungsgemäßen Verfahrens können in einer Verpackungseinheit bereitgestellt werden. Die Verpackungseinheit umfasst bevorzugt in einem ersten Kontainer ein oxidatives kosmetisches Mittel, enthaltend In einem kosmetischen Träger mindestens ein Oxidationsmittel und in einem zweiten Kontainer ein kosmetisches Mittel, enthaltend in einem kosmetischen Träger eine Pflanzenextraktkombination

Die Verpackungseinheit kann bevorzugt mindestens entweder einen Kontainer umfassen, der das Mittel des zweiten Erfindungsgegenstandes enthält, welches zusätzlich mindestens ein Oxidationsmittel enthält, oder kann mindestens zwei Kontainer umfassen, wobei ein erster Kontainer ein oxidatives kosmetisches Mittel, enthaltend in einem kosmetischen Träger mindestens ein Oxidationsmittel, enthält, ein zweiter Kontainer ein kosmetische Mittel, enthaltend in einem kosmetischen Träger die erfindungsgemäße weikstoffkombination, enthält und gegebenenfalls ein dritter Kontainer, der mindestens einen Bleichverstärker, eingebettet in einem kosmetischen Träger, enthält. Alle Kontainer können auch Kammern eines Mehrkammerbehältnisses sein.

Für den dritten Erfindungsgegenstand gilt *mutatis mutandis* das für den ersten und zweiten Erfindungsgegenstand Gesagte.

Die folgenden Beispiele sollen den Gegenstand der vorliegenden Erfindung erläutern ohne ihn jedoch zu beschränken.

Beispiele

Die folgenden Rezepturen wurden unter Anwendung bekannter Herstellungsverfahren bereitgestellt. Die angegebenen eingesetzten Mengen der Rohstoffe tragen, falls nicht anders gekennzeichnet, die Einheit Gewichtsprozent, bezogen auf das Gewicht der jeweiligen Zusammensetzungen. Es wurden nachstehende Handelsprodukte als Rohstoffe verwendet:

| | |
|---|---|
| Hydrenol® D | $C_{16}$-$C_{18}$-Fettalkohol (INCI-Bezeichnung: Cetearyl alcohol) (Cognis Deutschland) |
| Lorol® techn. | $C_{12}$-$C_{18}$-Fettalkohol (INCI-Bezeichnung: Coconut alcohol) (Cognis Deutschland) |
| Stenol® 16/18 | $C_{16-18}$-Fettalkohol (INCI-Bezeichnung: Cetearyl Alcohol) (Cognis) |
| Lorol® 16 | INCI-Bezeichnung: Cetyl Alcohol (Cognis) |
| Eumulgin® B1 | Cetylstearylakohol mit 12 EO-Einheiten (INCI-Bezeichnung: Ceteareth-12) (Cognis Deutschland) |
| Eumulgin® B2 | Cetylstearylalkohol mit ca. 20 EO-Einheiten (INCI-Bezeichnung: Ceteareth-20) (Cognis Deutschland) |
| Edenor® C14 | Myristinsäure (INCI-Bezeichnung: MyristicAcid) (Cognis) |
| Turpinal® SL | 1-Hydroxyethan-1,1-diphosphonsäure (INCI-Bezeichnung: Etidronic Acid, Aqua (Water)) (Solutia) |
| Plantapon® LGC | Alkylpolyglucosid-Carboxylat Natriumsalz; 30 % Aktivsubstanz (Cognis Deutschland) |
| Texapon® NSO UP | Natriumlaurylethersulfat (27 % Aktivsubstanz; INCI: Sodium Laureth Sulfate) (Hersteller: COGNIS) |
| Texapon® K 14 S 70 C | Laurylmyristylethersulfat-Natrium-Salz (ca. 68% bis 73% Aktivsubstanzgehalt'; INCI-Bezeichnung: Sodium Myreth Sulfate) (Cognis) |
| Disponil® FES 77 IS | Fettalkoholethersulfat (ca. 31-33% Aktivsubstanzgehalt in Wasser; INCI-Bezeichnung: Sodium Coceth-30 Sulfate) (Cognis) |
| Akypo® Soft 45 NV | 2-($C_{12-14}$-Fettalkoholethoxylat (4.5 EO))-essigsäure Natriumsalz; 21% Aktivsubstanz; INCI-Bezeichnung: Sodium Laureth-5 Carboxylate (KAO) |
| Gluadin® W 40 | Welzenproteinhydrolysat (mind. 40% Festkörper; INCI-Bezeichnung: Aqua (Water), Hydrolyzed Wheat Protein, Sodium Benzoate, Phenoxyethanol, Methylparaben, Propylparaben) (Cognls) |
| Plantacare® 1200 UP | $C_{12-16}$-Fettalkohol-1.4-glucosid (ca. 50-53% Aktivsubstanzgehalt; INCI-Bezeichnung: Lauryl Glucoside, Aqua (Water)) (Cognis) |
| Lamesoft® PO 65 | Alkylpolyglucosid Ölsäuremonoglycerid Gemisch (ca. 65-70% Festkörper; INCI-Bezeichnung: Coco-Glucoside, Glyceryl Oleate, Aqua (Water)) (Cognis) |
| Aculyn® 33 | 30 Gew.-% Aktivsubstanz in Wasser (INCI-Bezeichnung: Acrylates Copolymer) (Rohm & Haas) |
| DOW Corning® DB 110 | A nichtionogene Silikonemulsion (10 Gew.-% Aktivsubstanz) (INCI-Bezeichnung: Dimethicone) (Dow Corning) |
| Polymer® W 37194 | ca. 20Gew.-% Aktivsubstanzgehalt In Wasser; INCI-Bezeichnung: Acrylamidopropyltrimonlum Chloride/Acrylates Copolymer (Stockhausen) |

10 Bestimmung der Haarschädigung

Nachfolgende Rezepturen wurden nach einem Standardverfahren bereitgestellt (Mengenangaben sind jeweils Gew.-%). Sie entsprechen Färbecremes ohne Farbstoffvorprodukte.

Cremes:

| | T0 | T1 | T2 | T3 |
|---|---|---|---|---|
| Hydrenol D | 9,00 | 9,00 | 9,00 | 9,00 |
| Lorol | 3,00 | 3,00 | 3,00 | 3,00 |
| Eumulgin B1 | 0,50 | 0,50 | 0.50 | 0,50 |
| Eumulgin B2 | 0,50 | 0,50 | 0,50 | 0,50 |
| Isostearinsäure | 2,00 | 2,00 | 2,00 | 2,00 |
| Edenor C14 | 0,50 | 0,50 | 0,50 | 0,50 |
| Plantapon LGC | 5,00 | 5,00 | 5,00 | 5,00 |

(fortgesetzt)

| | T0 | T1 | T2 | T3 |
|---|---|---|---|---|
| Texapon NSO UP | 7,00 | 7,00 | 7,00 | 7,00 |
| Kaliumhydroxid 50% | 1,20 | 1,20 | 1,20 | 1,20 |
| Ammoniak 25% | 5,50 | 5,50 | 5,50 | 5,50 |
| Ascorbinsäure | 0,40 | 0,40 | 0,40 | 0,40 |
| Turpinal SL | 0,20 | 0,20 | 0,20 | 0,20 |
| Natriumsulfid | 0,50 | 0,50 | 0,50 | 0,50 |
| Natriumsilikat 40/42 | 0,50 | 0,50 | 0,50 | 0,50 |
| $\alpha$-Liponsäure | - | - | 0,50 | 0,50 |
| Extrapone Pueraria Root | - | 1,00 | - | 1,00 |
| Extrapone Sophora Flower | - | 0,30 | - | 0,30 |
| Wasser | ad.100 | ad.100 | ad.100 | ad.100 |

Entwickleremulsion:

| | E3 |
|---|---|
| Dipicolinsäure | 0,10 |
| Dinatriumpyrophosphat | 0,03 |
| Turpinal SL | 1,50 |
| Texapon NSO UP | 2,00 |
| Aculyn 33A | 12,00 |
| Wasserstoffperoxid 50% | 12,00 |
| Dow Corning DB 110 A | 0,07 |
| Ammoniak 25% | 0,62 |
| Wasser | ad. 100 |

Die Cremeformulierungen T0, T1, T2 und T3 wurden jeweils mit der Entwickleremulsion E3 im Gewichtsverhältnis 1:1 gemischt, Dabei ergibt sich im Valle von T-0 eine nicht erfindungsgemäße Mischung. Alle anderen resultierenden Anwendungsmischungen sind erfindungsgemäß.

Die Anwendungsmischung wurde im Gewichtsverhältnis 4:1 (Anwendungsmischung zu Haarmenge) für 30 min bei 32°C auf Haarsträhnen (Kerling Euro-Naturhaar 6/0) appliziert. Die Haarsträhnen wurden anschließend gewaschen und getrocknet. Der Vorgang wurde 3x wiederholt.

Die Haarschädigung im Rahmen der oben beschriebenen oxidativen Haarbehandlung wurde durch zwei unabhängige Methoden quantifiziert:

Methode 1: Aminosäureanalyse

Nach saurer Totalhydrolyse einer Probe der unbehandelten Haarsträhne und einer Probe der behandelten Haarsträhne wurde jeweils die Aminosäurezusammensetzung durch Ionenaustauschchromatographie bestimmt. Es ist bekannt, dass sich die oxidative Schädigung der Haarproteine Insbesondere in einer Bildung von Cysteinsäure aus Cystin manifestiert. Die Reduktion der Haarschädigung in % berechnet sich wiefolgt:

$$100 \cdot \left[ 1 - \frac{(\text{Cysteinsäuregehalt}_{\text{nach erfindungsgemäßer Anwendungsmischung}}) - (\text{Cysteinsäuregehalt}_{\text{unbehandeltes Haar}})}{(\text{Cysteinsäuregehalt}_{\text{Haar nach T0/E3}}) - (\text{Cysteinsäuregehalt}_{\text{unbehandeltes Haar}})} \right]$$

a) Experiment 1: Vergleich der Mischungen aus T0/E3 mit T1/E3

Bezogen auf den gesamten Aminosäuregehalt wurde im unbehandelten Haar ein Cysteinsäuregehalt von 0,56 mol-% gemessen. Dieser Wert stieg nach 3-maliger Behandlung mit T0/E3 auf 1,42 mol-%, mit T1/E3 dagegen nur auf 1,23 mol%.

b) Experiment 2: Vergleich der Mischungen aus T0/E3 mit T2/E3 und T3/E3

In einem weiteren Experiment wurde festgestellt, dass Extrakte aus den Wurzeln von Pueraria lobata (Extrapone Pueraria Root) und den Blüten von Sophora japonica (Extrapone Sophora flower) in Kombination mit alpha-Liponsäure einen synergistischen Schutz vor oxidativer Haarschädigung bieten. Bezogen auf den gesamten Aminosäuregehalt wurde hier im unbehandelten Haar ein Cysteinsäuregehalt von 0,49 mol-% gemessen. Dieser Wert stieg nach 3-maliger Behandlung mit T0/E3 auf 1,59 mol-%, mit T2/E3 auf 1,48 mol-% und mit T3/E3 nur auf 1,13 mol%.

Testergebnisse der Methode 1:

| Anwendungsmischung | Wirkstoffe | Reduktion der Haarschädigung [%] |
|---|---|---|
| T0/E3 | - | 0 |
| T1/E3 | 1,0% Extrapone Pueraria Root<br>0,3% Extrapone Sophora Flower | 13 |
| T2/E3 | 0,5% alpha-Liponsäure | 10 |
| T3/E3 | 0,5% alpha Liponsäure<br>1,0% Extrapone® Pueraria Root<br>0,3% Extrapone® Sophora Flower | 41 |

**Patentansprüche**

**1.** Kosmetische Verwendung einer Pflanzenextraktkombination aus

• Extrakt aus der Wurzel von *Pueraria lobata* und
• Extrakt aus der Blüte und/oder aus den Knospen der *Sophora japonica L.* mit $\alpha$-Liponsäure in Form der freien Säure und/oder der Salzform zur Haarpflege, insbesondere zur Verminderung der oxidativen Haarschädigung.

**2.** Mittel, enthaltend in einem kosmetischen Träger eine Pflanzenextraktkombination aus

• Extrakt aus der Wurzel von *Pueraria lobata* und
• Extrakt aus der Blüte und/oder aus den Knospen der *Sophora japonica L.* und zusätzlich $\alpha$-Liponsäure in Form der freien Säure und/oder der Salzform.

**3.** Mittel nach einem der Ansprüche 1 oder 2, **dadurch gekennzeichnet, daß** es jeden der miteinander kombinierten Pflanzenextrakte in einer Menge von 0,01 bis 5,0 Gew.-%, insbesondere von 0,1 bis 1,5 Gew.-%, jeweils bezogen auf das Gewicht des anwendungsbereiten Mittels, enthält.

**4.** Mittel nach einem der Ansprüche 1 bis 3, **dadurch gekennzeichnet, daß** es zusätzlich mindestens ein Oxidationsmittel enthält.

5. Mittel nach Anspruch 4, **dadurch gekennzeichnet, daß** das Oxidationsmittel Wasserstoffperoxyd und/oder mindestens ein Anlagerungsprodukt von Wasserstoffperoxyd an anorganische oder organische Verbindungen ist.

6. Mittel nach einem der Ansprüche 4 oder 5, **dadurch gekennzeichnet, daß** das Oxidationsmittel in einer Menge von 1,0 bis 10,0 Gew.-% bezogen auf das Gewicht des anwendungsbereiten Mittels, enthalten ist.

7. Mittel nach einem der Ansprüche 2 bis 6, **dadurch gekennzeichnet, daß** es zusätzlich mindestens eine farbverändernde Komponente enthält.

8. Mittel nach Anspruch 7, **dadurch gekennzeichnet, daß** die farbverändernde Komponente ausgewählt wird

(a) aus mindestens einem Oxidationsfarbstoffvorprodukt vom Typ der Entwicklerkomponenten und gegebenenfalls zusätzlich mindestens einer Kupplerkomponente
und/oder
(b) aus Oxofarbstoffvorprodukten
und/oder
(c) aus mindestens einem direktziehenden Farbstoff
und/oder
(d) aus mindestens einer Vorstufe naturanaloger Farbstoffe
und/oder
(e) aus mindestens einem Bleichverstärker.

9. Mittel nach einem der Ansprüche 2 bis 8, **dadurch gekennzeichnet, daß** es zusätzlich mindestens ein Tensid enthält.

10. Verfahren zur Haarbehandlung, in dem ein kosmetisches Mittel, enthaltend in einem kosmetischen Träger eine Pflanzenextraktkombination aus

• Extrakt aus der Wurzel von *Pueraria lobata* und
• Extrakt aus der Blüte und/oder aus den Knospen der *Sophora japonica L.*
und zusätzlich α-Liponsäure in Form der freien Säure und/oder der Salzform aufgetragen und gegebenenfalls nach einer Einwirkungszeit wieder abgespült wird.

11. Verfahren nach Anspruch 10, **dadurch gekennzeichnet, dass**

- ohne Zwischenspülen unmittelbar nach dem Auftragen des kosmetischen Mittels aus Anspruch 10 ein oxidatives kosmetisches Mittel, enthaltend in einem kosmetischen Träger mindestens ein Oxidationsmittel, aufgetragen und gegebenenfalls nach einer Einwirkungszeit wieder abgespült wird
und/oder
- das kosmetische Mittel gemäß Anspruch 10 zusätzlich mindestens ein Oxidationsmittel enthält.

12. Verpackungseinheit, umfassend in einem ersten Kontainer ein oxidatives kosmetisches Mittel, enthaltend in einem kosmetischen Träger mindestens ein Oxidationsmittel und in einem zweiten Kontainer ein kosmetisches Mittel, enthaltend in einem kosmetischen Träger eine Pflanzenextraktkombination aus

• Extrakt aus der Wurzel von *Pueraria lobata* und
• Extrakt aus der Blüte und/oder aus den Knospen der *Sophora japonica L.*
und zusätzlich α-Liponsäure in Form der freien Säure und/oder der Salzform

## Claims

1. A cosmetic use of a plant extract combination of

• extract from the root of *Pueraria lobata* and
• extract from the blossom and/or buds of *Sophora japonica L.*

with α-lipoic acid in the free acid and/or salt form, for hair care, in particular for reducing oxidative hair damage.

**2.** An agent, containing in a cosmetic carrier, a plant extract combination of

- extract from the root of *Pueraria lobata* and
- extract from the blossom and/or buds of *Sophora japonica L.*

and additionally α-lipoic acid in the free acid and/or salt form.

**3.** The agent according to any of claims 1 or 2, **characterized in that** it contains each of the plant extracts combined with each other in an amount from 0.01 to 5.0% by weight, in particular from 0.1 to 1.5% by weight, each based on the weight of the applicable agent.

**4.** The agent according to any of claims 1 to 3, **characterized in that** it further contains at least one oxidation agent.

**5.** The agent according to claim 4, **characterized in that** the oxidation agent is hydrogen peroxide and/or at least one addition product of hydrogen peroxide on inorganic or organic compounds.

**6.** The agent according to any of claims 4 or 5, **characterized in that** the oxidation agent is contained in an amount from 1.0 to 10.0% by weight, based on the weight of the applicable agent.

**7.** The agent according to any of claims 2 to 6, **characterized in that** it further contains at least one colour modifying component.

**8.** The agent according to claim 7, **characterized in that** the colour modifying component is selected

(a) from at least one oxidation dye precursor of the developer component type, and optionally further at least one coupling component
and/or
(b) from oxo dye precursors.
and/or
(c) from at least one substantive dye
and/or
(d) from at least one precursor of nature-analog dyes
and/or
(e) from at least one bleach booster.

**9.** The agent according to any of claims 2 to 8, **characterized in that** it further contains at least one surfactant.

**10.** A method for hair treatment, in which a cosmetic agent, containing in a cosmetic carrier a plant extract combination of

- extract from the root of *Pueraria lobata* and
- extract from the blossom and/or buds of *Sophora japonica L.*

and further α-lipoic acid in the free acid and/or salt form, is applied and is optionally rinsed off after a contact time.

**11.** The method according to claim 10, **characterized in that**

- without intermediate rinsing directly after the application of the cosmetic agent of claim 10, an oxidative cosmetic agent containing in a cosmetic carrier at least one oxidation agent is applied and is optionally rinsed off after a contact time and/or
- the cosmetic agent according to claim 10 further contains at least one oxidation agent.

**12.** A packaging unit, comprising in a first container an oxidative cosmetic agent, containing in a cosmetic carrier at least one oxidation agent and in a second container a cosmetic agent, containing in a cosmetic carrier, a plant extract combination of

- extract from the root of *Pueraria lobata* and
- extract from the blossom and/or buds of *Sophora japonica L.*

and additionally α-lipoic acid in the free acid and/or salt form.

## Revendications

1. Utilisation cosmétique d'une combinaison d'extraits de plantes à partir

   - d'un extrait de la racine de *Pueraria lobata,* et
   - d'un extrait des fleurs et/ou des bourgeons de *Sophora japonica L.,*

   avec de l'acide α-lipoïque sous la forme de l'acide libre et/ou de la forme saline
   pour les soins capillaires, en particulier pour diminuer la dégradation des cheveux par oxydation.

2. Agent contenant, dans un support cosmétique, une combinaison d'extraits de plantes à partir

   - d'un extrait de la racine de *Pueraria lobata,* et
   - d'un extrait des fleurs et/ou des bourgeons de *Sophora japonica L.,*

   et en outre de l'acide α-lipoïque sous la forme de l'acide libre et/ou de la forme saline.

3. Agent selon l'une quelconque des revendications 1 ou 2, **caractérisé en ce qu'**il contient chacun des extraits de plantes combinés l'un avec l'autre en une quantité de 0,01 à 5,0 % en poids, en particulier de 0,1 à 1,5 % en poids, chaque fois rapportés au poids de l'agent prêt à l'emploi.

4. Agent selon l'une quelconque des revendications 1 à 3, **caractérisé en ce qu'**il contient en outre au moins un agent d'oxydation.

5. Agent selon la revendication 4, **caractérisé en ce que** l'agent d'oxydation est le peroxyde d'hydrogène et/ou au moins un produit d'addition du peroxyde d'hydrogène sur des composés inorganiques ou organiques.

6. Agent selon l'une quelconque des revendications 4 ou 5, **caractérisé en ce qu'**il contient l'agent d'oxydation en une quantité de 1,0 à 10,0 % en poids, rapportés au poids de l'agent prêt à l'emploi.

7. Agent selon l'une quelconque des revendications 2 à 6, **caractérisé en ce qu'**il contient en outre au moins un composant modifiant les couleurs.

8. Agent selon la revendication 7, **caractérisé en ce que** le composant modifiant les couleurs est choisi

   (a) parmi au moins un précurseur de colorant d'oxydation du type des composants faisant office de développateurs et le cas échéant en outre au moins un composant faisant office de coupleur ;
   et/ou
   (b) parmi des précurseurs de colorants oxo ;
   et/ou
   (c) parmi au moins un colorant direct ;
   et/ou
   (d) parmi au moins un précurseur de colorants analogues à ceux que l'on trouve dans la nature ;
   et/ou
   (e) parmi au moins un renforçateur du blanchiment.

9. Agent selon l'une quelconque des revendications 2 à 8, **caractérisé en ce qu'**il contient en outre au moins un agent tensioactif.

10. Procédé pour le traitement capillaire, dans lequel on applique, un agent cosmétique contenant, dans un support cosmétique, une combinaison d'extraits de plantes à partir

    - d'un extrait de la racine de *Pueraria lobata,* et
    - d'un extrait des fleurs et/ou des bourgeons de *Sophora japonica L.,*

et en outre de l'acide α-lipoïque sous la forme de l'acide libre et/ou de la forme saline et on l'élimine à nouveau par rinçage, le cas échéant après l'avoir laissé agir pendant un certain temps.

**11.** Procédé selon la revendication 10, **caractérisé en ce que**

- en l'absence d'un rinçage intermédiaire, directement après l'application de l'agent cosmétique selon la revendication 10, on applique un agent cosmétique oxydant contenant, dans un support cosmétique, au moins un agent d'oxydation, et on l'élimine à nouveau par rinçage, le cas échéant après l'avoir laissé agir pendant un certain temps

et/ou

- l'agent cosmétique selon la revendication 10 contient en outre au moins un agent d'oxydation.

**12.** Unité de conditionnement comprenant, dans un premier récipient, un agent cosmétique oxydant contenant, dans un support cosmétique, au moins un agent d'oxydation et, dans un deuxième récipient, un agent cosmétique contenant, dans un support cosmétique, une combinaison d'extraits de plantes à partir

• d'un extrait de la racine de *Pueraria Iobata,* et
• d'un extrait des fleurs et/ou des bourgeons de *Sophora japonica L.,*
et en outre de l'acide α-lipoïque sous la forme de l'acide libre et/ou de la forme saline.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10229995 A1
- DE 29908573 U1
- EP 998908 A2
- DE 3725030 A
- DE 3723354 A
- DE 3926344 A
- DE 4440625 A1
- DE 19503465 A1
- GB 2104091 A
- EP 47714 A
- EP 217274 A
- EP 283817 A
- DE 2B17369
- DE 3929973
- DE OS19756454 A


### In der Beschreibung aufgeführte Nicht-Patentliteratur


- **Kh, Schrader.** Grundlagen und Rezepturen der Kosmetika. Hüthig Buch Verlag, 1989
- **Kh. Schrader.** Grundlagen und Rezepturen der Kosmetika. Hüthig Buch Verlag, 1989